Europäisches Patentamt

European Patent Office

Office européen des brevets

(19)

(11) **EP 0 639 646 B1**

(12) **EUROPEAN PATENT SPECIFICATION**

(45) Date of publication and mention
of the grant of the patent:
**06.05.1998 Bulletin 1998/19**

(51) Int Cl.[6]: **C12Q 1/32**

(86) International application number:
**PCT/JP91/01743**

(21) Application number: **92901435.5**

(22) Date of filing: **20.12.1991**

(87) International publication number:
**WO 92/20817 (26.11.1992 Gazette 1992/29)**

(54) **HIGH PRECISION DETERMINATION OF D-GLUCOSE-6-PHOSPHATE AND COMPOSITION THEREFOR**

HOCHPRÄZISIONSBESTIMMUNG VON GLUCOSE-6-PHOSPHAT UND DAFÜR GEEIGNETE ZUSAMMENSETZUNG

DETERMINATION TRES PRECISE D'UNE QUANTITE DE D-GLUCOSE-6-PHOSPHATE, ET COMPOSITION A CET EFFET

(84) Designated Contracting States:
**DE FR IT**

(30) Priority: **14.05.1991 JP 138324/91**

(43) Date of publication of application:
**22.02.1995 Bulletin 1995/08**

(73) Proprietor: **ASAHI KASEI KOGYO KABUSHIKI KAISHA**
**Osaka (JP)**

(72) Inventors:
• **UEDA, Shigeru, 1421-237, Nanjo, Nirayama-cho**
  **Shizuoka 410-21 (JP)**
• **TAKAHASHI, Mamoru, 684-1, Kakita,**
  **Shimizu-cho**
  **Shizuoka 411 (JP)**
• **MISAKI, Hideo, 839-1, Mifuku, Ohito-cho**
  **Shizuoka 410-23 (JP)**
• **IMAMURA, Shigeyuki, 236-2, Mifuku, Ohito-cho**
  **Shizuoka 410-23 (JP)**

(74) Representative: **Boeters, Hans Dietrich, Dr.**
  **Patentanwälte Boeters & Bauer,**
  **Bereiteranger 15**
  **81541 München (DE)**

(56) References cited:
**EP-A- 0 293 732      EP-A- 0 477 001**
**EP-A- 0 575 610**

• **ANALYTICAL BIOCHEMISTRY, vol. 182, no. 2, November 1989, pages 399-404, XP000574490 FLORINI, J.R.: "Assay of creatine kinase in microtiter plates using thio-NAD to allow monitoring at 405 nm."**
• **SHADAN HOJIN NIHON SEIKAGAKU-KAI (author), "Biochemical Experiment Lecture 5 Enzyme Research (upper)", August 20, 1975 (20.08.75), Tokyo Kagaku Dojin, p. 121-135.**

## Description

<u>Technical field</u>

The present invention relates to a novel method for the quantitative determination of glucose-6-phosphate utilizing an enzymatic cycling reaction. The present invention also relates to a novel analytical reagent for use in the above-mentioned quantitative determination.

<u>Background Art</u>

In recent years, in the analysis of biological samples for the purpose of clinical diagnosis, an enzymatic method has increasingly been adopted in place of a chemical method because the enzymatic method is superior to the chemical method in specificity for chemical substances to be detected. Among the enzymes which are widely used in the enzymatic analysis is glucose-6-phosphate dehydrogenase (EC 1.1.1.49), which catalyzes the following reversible reaction.

$$\text{D-glucose-6-phosphate} + \text{NAD(P)}^+ \rightleftarrows$$

$$\text{D-glucono-}\delta\text{-lactone-6-phosphate} + \text{NAD(P)H} + \text{H}^+$$

This enzyme is used in combination with other enzymes for the quantitative determination of ATP, glucose and the like and for the coupling assay of enzymes, such as creatine kinase and the like. For example, in the quantitative determination of glucose, glucose is reacted with hexokinase (EC 2.7.1.1) in the presence of ATP to thereby produce D-glucose-6-phosphate, and the produced D-glucose-6-phosphate is then reacted with D-glucose-6-phosphate dehydrogenase in the presence of NAD(P) to produce D-glucono-$\delta$-lactone-6-phosphate and reduced NAD(P). In this case, the amount of the reduced NAD(P) produced by the above reaction is, generally, spectrophotometrically measured in terms of an increase in absorbance at 340 nm. Alternatively, it has been proposed to measure the amount of the reduced NAD(P) by a method in which the reduced NAD(P) is reacted with diaphorase or nitroblue tetrazolium to thereby produce a formazan pigment and the amount of the produced formazan pigment is then analyzed. This pigment, however, is insoluble and, therefore, this method in which the reduced NAD(P) is converted to a pigment is unsuitable for use in automatic analysis. Another method has also been reported in which thio-NADP is employed in place of NADP (Extra-edition of Journal of Medical Technology, Vol. 22, p. 1232, Japan, 1978). This method using thio-NADP has a sensitivity which is about twice those of the conventional methods.

In addition to the above applications, the glucose-6-phosphate dehydrogenase, which has a wide variety of applications in the field of clinical diagnosis, is used for such applications as the measurement of the activity of $\alpha$-amylase (EC 3.2.1.1), phosphoglucomutase or phosphohexose isomerase (EC 5.3.1.9); the quantitative determination of D-sorbitol or D-fructose; and so on.

Further, the determination of inorganic phosphorus has been conducted by reacting it with an enzymatic reagent comprising sucrose phosphorylase and phosphoglucomutase to thereby produce D-glucose-6-phosphate, and measure the amount of the produced D-glucose-6-phosphate, so that the amount of inorganic phosphorous is determined therefrom (Journal of Clinical Laboratory Instruments and Reagents, Vol. 13, p. 1137-1143, Japan, 1990). Still further, in the determination of D-fructose, it has been proposed to react D-fructose with hexokinase and phosphoglucose isomerase to produce D-glucose-6-phosphate, and measure the produced amount of the D-glucose-6-phosphate, so that the amount of D-fructose is determined therefrom. This method, however, has a problem in that substrates for hexokinase include not only D-fructose but also D-glucose. To solve this problem, another method has been reported for the determination of the D-fructose in which fructokinase which is specific for D-fructose is utilized (Anal. Biochem., Vol. 54, p.205, 1973). The ordinary concentration of D-fructose in blood serum is as low as about 40 $\mu$m mol/l. It has been reported that the D-fructose concentration is increased in diabetes patients [Nippon Rinsho Zokan-go (Extra-edition of Japanese Journal of Clinical Medicine), Vol. 47, p. 468, 1989] and, therefore, a highly sensitive determination method for D-fructose has been desired.

Generally, in an analysis utilizing an enzymatic reaction, a target chemical substance to be determined is subjected to an enzymatic reaction to produce a spectrophotometrically detectable substance, such as hydrogen peroxide, reduced NAD(P) or the like in a stoichiometrical amount relative to the amount of the target chemical substance. At present, for the determination of such a detectable substance, a method using a spectrophotometer has been most widely adopted. This method, however, has defects in that the sensitivity is limited to low levels and, therefore, this method is unsatisfactory when the quantity of a target chemical substance to be detected is low.

To solve the above problems, when the target chemical substance content of a biological sample is small or the quantity of a biological sample containing the target chemical substance is small, fluorescence analysis and luminescence analysis have widely been adopted because fluorescence and luminescence analyses are superior in sensitivity to spectrophotometric analysis. These methods, however, are unsuitable for wide use in various fields, such as clinical diagnosis since apparatus to be used in these methods are not easily available. As a method for the determination of a very small quantity of a substance which can be converted to, for example, an equimolar amount of a coenzyme relative to the amount of the substance, the so-called enzymatic cycling method has also been known, in which two types of enzymes are simultaneously utilized so that the sensitivity for the coenzyme can be greatly amplified. For example, the NAD cycling method, CoA cycling method, ATP cycling method and the like have been known. These methods, however, have been almost not employed in routine analyses, such as clinical diagnosis, etc., because the procedures involved in these methods are cumbersome.

A highly sensitive determination method has various advantages. For example, a highly sensitive determination method ensures accuracy in the determination of a target chemical substance even when the quantity of the chemical substance is small. In addition, the quantity of a biological sample to be used can be reduced. Therefore, with respect to biological samples containing various other substances in addition to a target substance as in the case of blood serum, a highly sensitive determination method is effective for minimizing the influence of non-target substances present in the biological sample on the determination of the target substance because the quantity of the biological sample can be reduced to a minimum. Also, by virtue of the high sensitivity of the method, even with a limited quantity of a biological sample, the number of the analysis items can be increased. Furthermore, the amount of a biological sample to be collected can be reduced, so that when the biological sample is human blood, a psychological burden on the donor can be reduced. Moreover, the amount of waste biological materials can be minimized, so that the problem of the environmental pollution caused by such waste materials can be eliminated.

Task to be Solved by the Invention

As mentioned above, analytical methods using glucose-6-phosphate dehydrogenase have been reported not only for the determination of various substances but also for the measurement of the activities of various enzymes. Any of those methods, however, is not highly sensitive. In these situations, a simple and highly sensitive method has been desired, particularly for the determination of a small quantity of a chemical substance and for the measurement of the activity of a small quantity of an enzyme. If D-glucose can be determined with high sensitivity by a highly sensitive method in which the use of hexokinase is combined, it is expected that such a method can also be applied to the determination of the activity of β-galactosidase which is frequently used as a marker enzyme in enzyme immunoassay.

Means to Solve the Task

From the above context, the present inventors have made extensive and intensive studies with a view toward solving the above-mentioned problems accompanying the conventional methods for the quantitative determination of chemical substances in biological samples. As a result, they have found that in the reversible enzymatic reaction (producing D-glucono-δ-lactone-6-phosphate from D-glucose-6-phosphate) which is catalyzed by a glucose-6-phosphate dehydrogenase, a cycling reaction in which the following two types of coenzymes are simultaneously used: (i) a first coenzyme selected from the group consisting of thionicotinamide adenine dinucleotide or its analogue (thio-NAD compound) and thionicotinamide adenine dinucleotide phosphate or its analogue (thio-NADP compound) and (ii) a second coenzyme selected from the group consisting of nicotinamide adenine dinucleotide or its analogue (NAD compound) and nicotinamide adenine dinucleotide phosphate or its analogue (NADP compound), can be advantageously, effectively utilized for the quantitative determination of D-glucose-6-phosphate in a biological sample. That is, the target chemical substance, i.e., D-glucose-6-phosphate can be quantitatively determined by measuring a change in amount of $A_2$ or $B_1$, which is caused for a predetermined period of time during the cycling reaction represented by the following formula (I):

EP 0 639 646 B1

$$A_1 \qquad \text{Glucose-6-} \qquad A_2$$
Glucose-6-
phosphate
Dehydrogenase

D-Glucose-6-phosphate $\leftarrow \qquad \rightarrow$ D-Glucono-δ-lactone-
6-phosphate

(I)

$$B_2 \qquad \qquad B_1$$

wherein $A_1$ is a thio-NADP compound, a thio-NAD compound, an NADP compound or an NAD compound; $A_2$ is a reduced form of $A_1$; $B_1$ is a reduced NADP compound or a reduced NAD compound when $A_1$ is a thio-NADP compound or a thio-NAD compound, or a reduced thio-NADP compound or a reduced thio-NAD compound when $A_1$ is an NADP compound or an NAD compound; and $B_2$ is an oxidized form of $B_1$. Further, the present inventors have also found that the change in amount of coenzyme $A_2$ or $B_1$ can be easily measured in terms of the change in absorbance at a wavelength specific for coenzyme $A_2$ or $B_1$ with respect to the reaction mixture (actually, in terms of the difference in absorbance at a specific wavelength, which is observed with respect to the reaction mixture as between predetermined two time points during the above-mentioned cycling reaction) because coenzymes $A_2$ and $B_1$ are different in absorption maximum from each other (a reduced thio-NAD compound and a reduced thio-NADP compound exhibit an absorption maximum at about 400 nm, and a reduced NAD compound and a reduced NADP compound exhibit an absorption maximum at about 340 nm). Thus, a highly sensitive method for the quantitative determination of D-glucose-6-phosphate, which can be simply, efficiently carried out, has been realized. Based upon the above findings, the present invention has been completed.

Brief Description of The Drawings

In the drawings:

Fig. 1 is a graph showing the results of the rate assay of D-glucose-6-phosphate at a wavelength of 400 nm conducted in Example 1;
Fig. 2 is a graph showing the results of the rate assay of D-glucose-6-phosphate at a wavelength of 400 nm conducted in Example 2;
Fig. 3 is a graph showing the results of the rate assay of D-glucose-6-phosphate at a wavelength of 400 nm conducted in Example 3; and
Fig. 4 is a graph showing the results of the rate assay of D-fructose at a wavelength of 400 nm conducted in Example 4.

Disclosure of The Invention

According to the present invention, there is provided a method for the quantitative determination of D-glucose-6-phosphate, which comprises:
reacting a biological sample containing D-glucose-6-phosphate with a reagent comprising:

(1) a glucose-6-phosphate dehydrogenase which catalyzes the reversible reaction producing D-glucono-δ-lactone-6-phosphate from D-glucose-6-phosphate as a substrate in the presence of (i) a first coenzyme selected from the group consisting of thionicotinamide adenine dinucleotide phosphate or its analogue (thio-NADP compound) and thionicotinamide adenine dinucleotide or its analogue (thio-NAD compound) and (ii) a second coenzyme selected from the group consisting of nicotinamide adenine dinucleotide phosphate or its analogue (NADP compound) and nicotinamide adenine dinucleotide or its analogue (NAD compound);
(2) $A_1$ (defined hereinbelow); and
(3) $B_1$ (defined hereinbelow):

the components (1), (2) and (3) participating in the following cycling reaction:

4

$$A_1 \quad \text{Glucose-6-phosphate Dehydrogenase} \quad A_2$$

D-Glucose-6-phosphate $\rightleftarrows$ D-Glucono-$\delta$-lactone-6-phosphate

$$B_2 \qquad B_1$$

(I)

wherein $A_1$ is a thio-NADP compound, a thio-NAD compound, an NADP compound or an NAD compound; $A_2$ is a reduced form of $A_1$; $B_1$ is a reduced NADP compound or a reduced NAD compound when $A_1$ is a thio-NADP compound or a thio-NAD compound, or a reduced thio-NADP compound or a reduced thio-NAD compound when $A_1$ is an NADP compound or an NAD compound; and $B_2$ is an oxidized form of $B_1$;
thereby effecting the cycling reaction; and
measuring a change in amount of $A_2$ or $B_1$.

In another aspect of the present invention, there is provided an analytical reagent for use in the quantitative determination of D-glucose-6-phosphate, which comprises components (1), (2) and (3) as defined above.

The glucose-6-phosphate dehydrogenase (EC 1.1.1.49) to be used in the method of the present invention is defined as a dehydrogenase which catalyzes reversible reactions producing D-glucono-$\delta$-lactone-6-phosphate from D-glucose-6-phosphate as a substrate in the presence of (i) a first coenzyme selected from the group consisting of a thio-NADP compound and a thio-NAD compound and (ii) a second coenzyme selected from an NADP compound and an NAD compound. A typical example of the above-mentioned reversible reactions is represented by the following formula:

$$\text{D-glucose-6-phosphate} + \text{NAD(P)}^+ \rightleftarrows$$

$$\text{D-glucono-}\delta\text{-lactone-6-phosphate} + \text{NAD(P)H} + \text{H}^+.$$

Such a glucose-6-phosphate dehydrogenase is present widely in living bodies and plants, and is purified from mammary gland tissue, red cells, livers, plants and microorganisms and the like. A glucose-6-phosphate dehydrogenase is generally specifically reactive to NADP as a coenzyme. Examples of NADP-specific glucose-6-phosphate dehydrogenases are those derived form, e.g., yeast. However, glucose-6-phosphate dehydrogenases derived from, for example, Leuconostoc mesenteroides, Azotobacter vinelandii and Pseudomonas fluorescens, are reactive to not only NADP but also NAD (Enzyme Handbook, p.20 - 21, Asakura Shoten, Japan, 1983).

Glucose-6-phosphate dehydrogenases derived from Bacillus stearothermophilus are also reactive to both NAD and NADP (Biochemistry, vol. 52, p819, 1980). Among these dehydrogenases, a glucose-6-phosphate dehydrogenase derived from yeast or Leuconostoc mesenteroides is sold by, for example, Oriental Yeast Industries Co., Ltd., Japan, Boehringer Mannheim GmbH, Germany, and Sigma Co., Ltd., U.S.A. For example, with respect to glucose-6-phosphate dehydrogenase derived from baker's yeast (which is sold by Sigma Co., Ltd., U.S.A.), when the activity exerted with use of NADP in 40 mM Tris-HCl buffer (pH 7.1) is taken as 100 %, the relative activity exerted with use of thio-NADP in the same buffer is 67 % and no activity was observed with use of NAD.

With respect to glucose-6-phosphate dehydrogenase derived from Leuconostoc mesenteroides (which is sold by Toyobo Co., Ltd., Japan), when the activity exerted with use of NADP is taken as 100 %, the relative activities exerted with uses of thio-NADP, NAD and thio-NAD are 27 %, 176 % and 49 %, respectively. With respect to glucose-6-phosphate dehydrogenase derived from Bacillus sp. (which is sold by Asahi Kasei Kogyo Kabushiki Kaisha, Japan), when the activity exerted with use of NADP in 40 mM phosphate buffer (pH 7.0) is taken as 100 %, the relative activities exerted with uses of thio-NADP, NAD and thio-NAD in the same buffer are 40 %, 7 % and 34 %, respectively. Other glucose-6-phosphate dehydrogenases of other origins also can be used in appropriate systems.

With respect to the reactivity of glucose-6-phosphate dehydrogenases to coenzymes, i.e., an NAD(P) compound and a thio-NAD(P) compound, there is no particular limitation as long as the glucose-6-phosphate dehydrogenase employed catalyzes the reversible reaction producing D-glucono-δ-lactone-6-phosphate from D-glucose-6-phosphate as a substrate with the aid of the coenzymes. The reactivity can be confirmed using these coenzymes and D-glucose-6-phosphate as a substrate as well as the glucose-6-phosphate dehydrogenases.

In the present invention, coenzymes $A_1$ and $B_2$ are appropriately selected from the group consisting of a thio-NADP compound, a thio-NAD compound, an NADP compound and an NAD compound in accordance with the designed reaction scheme. Examples of thio-NADP compounds and thio-NAD compounds include thionicotinamide adenine dinucleotide phosphate (thio-NADP) and thionicotinamide hypoxanthine dinucleotide phosphate; and thionicotinamide adenine dinucleotide (thio-NAD) and thionicotinamide hypoxanthine dinucleotide. Examples of NADP compounds and NAD compounds include nicotinamide adenine dinucleotide phosphate (NADP), acetylpyridine adenine dinucleotide phosphate (acetyl NADP), acetylpyridine hypoxanthine dinucleotide phosphate, nicotinamide hypoxanthine dinucleotide phosphate (deamino NADP); and nicotinamide adenine dinucleotide (NAD), acetylpyridine adenine dinucleotide (acetyl NAD), acetylpyridine hypoxanthine dinucleotide and nicotinamide hypoxanthine dinucleotide (deamino NAD). Hereinafter, reduced types of these coenzymes are referred to, for example, as a thio-NADPH compound, a thio-NADH compound, an NADPH compound and an NADH compound.

In the present invention, for example, when $A_1$ is a thio-NAD(P) compound, $B_1$ is an NAD(P)H compound, and when $A_1$ is an NAD(P) compound, $B_1$ is a thio-NAD(P)H compound.

When the glucose-6-phosphate dehydrogenase to be used is capable of reacting with only NAD type coenzymes, i.e., a thio-NAD compound and an NAD compound, appropriate NAD type coenzymes are selected from the above-mentioned thio-NAD compounds and NAD compounds. When the glucose-6-phosphate dehydrogenase to be used is capable of reacting with only NADP type coenzymes, i.e., a thio-NADP compound and an NADP compound, appropriate NADP coenzymes are selected from the above-mentioned thio-NADP compounds and NADP compounds. When the glucose-6-phosphate dehydrogenase to be used is capable of reacting with either NAD type coenzymes or NADP type coenzymes, appropriate NAD type or NADP type coenzymes are selected from the above-mentioned thio-NAD compounds, thio-NADP compounds, NAD compounds and NADP compounds. In practice, an appropriate oxidized form or reduced form thereof is used in the method of the present invention.

According to the highly sensitive method of the present invention, it is possible to accurately determine D-glucose-6-phosphate which is originally present in such a form in biological samples.

The method of the present invention has other utilities. That is, it is possible not only to determine a substrate in an enzymatic reaction system producing D-glucose-6-phosphate, but also measure the activity of an enzyme involved in such an enzymatic reaction system. Further, according to the highly sensitive method of the present invention, it is possible not only to determine a substrate in a first enzymatic reaction system which comprises one or more reaction steps and can be linked to a second enzymatic reaction system producing D-glucose-6-phosphate, but also measure the activity of an enzyme involved in the first enzymatic reaction system. Illustrative examples of such utilities are described below.

(1) Enzymatic reaction of hexokinase (EC 2.7.1.1) with ATP and D-glucose as substrates:

$$\text{D-glucose} + \text{ATP} \rightarrow \text{D-glucose-6-phosphate} + \text{ADP}$$

In the system involving the above reaction, by determining D-glucose-6-phosphate produced, the reactants and enzyme involved in the above reaction can be measured. That is, in the system involving the above reaction, by determining D-glucose-6-phosphate produced, not only can ATP or D-glucose be determined, but also the activity of hexokinase can be measured.

(2) Enzymatic reaction of phosphoglucose isomerase (EC 5.3.1.9) with D-fructose-6-phosphate as a substrate:

$$\text{D-fructose-6-phosphate} \rightarrow \text{D-glucose-6-phosphate}$$

In the system involving the above reaction, by determining D-glucose-6-phosphate produced, the reactant and enzyme involved in the above reaction can be measured.

(3) Enzymatic reaction of phosphoglucomutase (EC 2.7.5.1) with D-glucose-1-phosphate and D-glucose-1,6-diphosphate as substrates:

$$\text{D-glucose-1-phosphate} + \text{D-glucose-1,6-diphosphate}$$

$$\rightarrow \text{D-glucose-6-phosphate} + \text{D-glucose-1,6-diphosphate}$$

In the system involving the above reaction, by determining D-glucose-6-phosphate produced, the reactants and enzyme involved in the above reaction can be measured.

(4) Enzymatic reaction of item (2) above, wherein D-fructose-6-phosphate is produced from D-fructose-1,6-diphosphate and inorganic phosphorus by the following enzymatic reaction catalyzed by 6-phosphofructokinase (EC 2.7.1.90):

$$\text{D-fructose-1,6-diphosphate + inorganic phosphorus}$$

$$\rightarrow \text{D-fructose-6-phosphate + pyrophosphate}$$

In the system involving the above reaction, by determining D-glucose-6-phosphate produced, the reactants and enzymes involved in the above reaction can be measured.

(5) Enzymatic reaction of item (2) above, wherein D-fructose-6-phosphate is produced from D-fructose and ATP by the following enzymatic reaction catalyzed by fructokinase (EC 2.7.1.4) or hexokinase (EC 2.7.1.1):

$$\text{D-fructose + ATP} \rightarrow \text{D-fructose-6-phosphate + ADP}$$

In the system involving the above reaction, by determining D-glucose-6-phosphate produced, the reactants and enzymes involved in the above reaction can be measured.

(6) Enzymatic reaction of item (3) above, wherein D-glucose-1-phosphate is produced from glycogen and inorganic phosphorus by the following enzymatic reaction catalyzed by glycogen phosphorylase (EC 2.4.1.1):

$$(\text{glycogen})_n \text{ + inorganic phosphorus} \rightarrow \text{D-glucose-1-}$$

$$\text{phosphate + } (\text{glycogen})_{n-1}$$

In the system involving the above reaction, by determining D-glucose-6-phosphate produced, the reactants and enzymes involved in the above reaction can be measured.

(7) Enzymatic reaction of item (3) above, wherein D-glucose-I-phosphate is produced from sucrose and inorganic phosphorus by the following enzymatic reaction catalyzed by sucrose phosphorylase (EC 2.4.1.7):

$$\text{sucrose + inorganic phosphorus} \rightarrow \text{D-glucose-1-}$$

$$\text{phosphate + D-fructose}$$

In the system involving the above reaction, by determining D-glucose-6-phosphate produced, the reactants and enzymes involved in the above reaction can be measured.

(8) Enzymatic reaction of item (1) above, wherein ATP is produced from creatine phosphate and ADP by the following enzymatic reaction catalyzed by creatine kinase (EC 2.7.3.2):

$$\text{creatine phosphate + ADP} \rightarrow \text{creatine + ATP}$$

In the system involving the above reaction, by determining D-glucose-6-phosphate produced, the reactants and enzymes involved in the above reaction can be measured.

(9) Enzymatic reaction of item (1) above, wherein ATP is produced from phosphoenolpyruvic acid and ADP by the following enzymatic reaction catalyzed by pyruvate kinase (EC 2.7.1.40):

$$\text{phosphoenolpyruvic acid + ADP} \rightarrow \text{pyruvic acid + ATP}$$

In the system involving the above reaction, by determining D-glucose-6-phosphate produced, the reactants and enzymes involved in the above reaction can be measured.

(10) Enzymatic reaction of item (1) above, wherein ATP is produced from acetyl phosphate and ADP by the following enzymatic reaction catalyzed by acetate kinase (EC 2.7.2.1):

$$\text{acetyl phosphate} + \text{ADP} \rightarrow \text{acetic acid} + \text{ATP}$$

In the system involving the above reaction, by determining D-glucose-6-phosphate produced, the reactants and enzymes involved in the above reaction can be measured.

(11) Enzymatic reaction of item (5) above, wherein D-fructose is produced from D-sorbitol and $NAD^+$ by the following enzymatic reaction catalyzed by sorbitol dehydrogenase (EC 1.1.1.14)

$$\text{D-sorbitol} + \text{NAD}^+ \rightarrow \text{D-fructose} + \text{NADH} + \text{H}^+$$

In the system involving the above reaction, by determining D-glucose-6-phosphate produced, the reactants and enzymes involved in the above reaction can be measured.

(12) Enzymatic reaction of item (1) above, wherein D-glucose is produced from $\beta$-lactose and $H_2O$ by the following enzymatic reaction catalyzed by $\beta$-D-galactosidase (EC 3.2.1.23):

$$\beta\text{-lactose} + \text{H}_2\text{O} \rightarrow \text{D-galactose} + \text{D-glucose}$$

In the system involving the above reaction, by determining D-glucose-6-phosphate produced, the reactants and enzymes involved in the above reaction can be measured.

In the method of the present invention, component (2) (coenzyme $A_1$) and component (3) (coenzyme $B_1$) are used in excess amounts relative not only to the amount of D-glucose-6-phosphate, but also the respective Km (Michaelis constant) values of component (1) (glucose-6-phosphate dehydrogenase) for components (2) and (3). It is especially preferred to use components (2) and (3) individually in amounts of from 20 to 10,000 moles per mole of D-glucose-6-phosphate.

As mentioned above, in another aspect of the present invention, there is provided an analytical reagent for use in the above-mentioned quantitative determination. In the analytical reagent of the present invention, the concentration of each of component (2) ($A_1$) and component (3) ($B_1$) is 0.02 to 100 mM, preferably 0.05 to 20 mM, and the concentration of component (1) (glucose-6-phosphate dehydrogenase) is 1 to 1,000 u/ml, preferably 2 to 400 u/ml. However, an appropriate concentration of each of components (1) (2) and (3) in the analytical reagent is varied depending on the type of a biological sample to be tested and, if desired, these components can be used in larger amounts.

In the present invention, when $B_2$ functions also as a coenzyme for a certain dehydrogenase other than the glucose-6-phosphate dehydrogenase of the cycling reaction (I), which certain dehydrogenase does not react with D-glucose-6-phosphate but acts to advance the reaction for converting $B_2$ to $B_1$ in cooperation with a substrate for the certain dehydrogenase, such a certain dehydrogenase (which is hereinafter frequently referred to as "second dehydrogenase") can also be additionally incorporated together with a substrate therefor (second dehydrogenase is referred to as "component (4)") into the reagent for effecting the following cycling reaction (II):

$$A_1 \quad \text{Glucose-6-phosphate Dehydrogenase} \quad A_2$$

D-Glucose-6-phosphate $\longleftarrow$ $\longrightarrow$ D-Glucono-$\delta$-lactone-6-phosphate

(II)

$$B_2 \qquad B_1$$

Second Dehydrogenase

wherein $A_1$ is a thio-NADP compound, a thio-NAD compound, an NADP compound or an NAD compound; $A_2$ is a reduced form of $A_1$; $B_1$ is a reduced NADP compound or a reduced NAD compound when $A_1$ is a thio-NADP compound or a thio-NAD compound, or a reduced thio-NADP compound or a reduced thio-NAD compound when $A_1$ is a NADP compound or an NAD compound; and $B_2$ is an oxidized form of $B_1$.

In the above reaction system, as the second dehydrogenase, a dehydrogenase which does substantially not react with $A_1$ but acts to advance the reaction for converting $B_2$ to $B_1$, is chosen. Alternatively, by choosing reaction conditions under which the second dehydrogenase does not react with $A_1$, the objective can be attained. For example, there can be chosen appropriate $A_1$-$B_2$ amount relationship conditions under which the second dehydrogenase does substantially not react with $A_1$. The quantitative determination of the target chemical substance participating in reaction (II), can be achieved by measuring a change in the amount of $A_2$ [which is caused for a predetermined period of time during reaction (II)].

The second dehydrogenase can be advantageously used in order to regenerate $B_1$, so that the amount of $B_1$ to be used in the analytical reaction can be reduced. This is particularly useful when $B_1$ is an expensive compound. It is also possible to use $B_2$ alone or a mixture of $B_1$ and $B_2$ at the initiation of the reaction. For conducting reaction (II), an amount of at least one coenzyme selected from $B_1$ and $B_2$ is preferably not larger than 1/10 mole per mole of $A_1$, although the amount is not particularly limited.

In practicing the above method for quantitative determination of the present invention using a second dehydrogenase as component (4), $A_1$ is used in a concentration of 0.02 to 100 mM, preferably 0.05 to 20 mM. $B_2$ and/or $B_1$ is used in a concentration of 0.05 to 5,000 µM, preferably 5 to 500 µM. A glucose-6-phosphate dehydrogenase as the first dehydrogenase is used in a concentration of 1 to 1000 u/ml, preferably 2 to 400 u/ml. The concentration of the second dehydrogenase (u/ml) can be 20 times or more the Km value (unit: mM) thereof for $B_2$, e.g., 1 to 100 u/ml. The substrate for the second dehydrogenase can be used in a stoichiometrically excess amount, for example, 0.05 to 20 mM. The amounts of the components of the reagent for the cycling reaction can be varied depending on the type of a biological sample to be tested. The amount exceeding the above can also be employed.

As examples of combinations of second dehydrogenases and substrates therefor, the following combinations can be mentioned. When $B_2$ is an NAD compound or a thio-NAD compound, there can be mentioned combinations of: alcohol dehydrogenase (EC 1.1.1.1) and ethanol; glycerol dehydrogenase (EC 1.1.1.6) derived from E. coli and glycerol; L-glycerol-3-phosphate dehydrogenase (EC 1.1.1.8) derived from rabbit muscle and L-glycerol-3-phosphate; malate dehydrogenase (EC 1.1.1.37) derived from pig or bovine heart and L-malic acid; and glyceraldehyde phosphate dehydrogenase (EC 1.1.1.12) derived from rabbit muscle, liver, yeast or E. coli, D-glyceraldehyde phosphate and phosphoric acid. When $B_2$ is an NADP compound or a thio-NADP compound, there can be mentioned combinations of: isocitrate dehydrogenase (EC 1.1.1.42) derived from yeast or pig heart and iso-citric acid; glyoxylate dehydrogenase (EC 1.2.1.17) derived from Pseudomonas oxalaticus, CoA and glyoxylic acid; phosphogluconate dehydrogenase (EC 1.1.1.44) derived from rat liver, beer yeast or E. coli and 6-phospho-D-gluconic acid; glyceraldehyde phosphate dehydrogenase (EC 1.2.1.13) derived from plant chlorophyll, D-glyceraldehyde-3-phosphate and phosphoric acid; and benzaldehyde dehydrogenase (EC 1.2.1.7) derived from Pseudomonas fluorescens and benzaldehyde.

Furthermore, in the present invention, when $A_2$ functions also as a coenzyme for a certain dehydrogenase other than the glucose-6-phosphate dehydrogenase of the reaction (I) and the second dehydrogenase of the reaction (II), which certain dehydrogenase does not react with D-glucose-6-phosphate but acts to advance the reaction for converting $A_2$ to $A_1$ in cooperation with a substrate for the certain dehydrogenase, such a certain dehydrogenase (which is hereinafter frequently referred to as "third dehydrogenase") can also be additionally incorporated together with a substrate therefor (third dehydrogenase is referred to as "component (5)") into the reagent for effecting the following cycling reaction (III):

wherein $A_1$ is a thio-NADP compound, a thio-NAD compound, an NADP compound or an NAD compound; $A_2$ is a reduced form of $A_1$; $B_1$ is a reduced NADP compound or a reduced NAD compound when $A_1$ is a thio-NADP compound or an thio-NAD compound, or a reduced thio-NADP compound or a reduced thio-NAD compound when $A_1$ is a NADP compound or an NAD compound; and $B_2$ is an oxidized form of $B_1$.

In the above reaction system, as the third dehydrogenase, a dehydrogenase which does substantially not react with $B_1$, but acts to advance the reaction for converting $A_2$ to $A_1$, is chosen. Alternatively, by choosing reaction conditions under which the third dehydrogenase does not react with $B_1$, the objective can be attained. For example, there can be chosen appropriate $B_1$-$A_2$ amount relationship conditions under which the third dehydrogenase does substantially not react with $B_1$. The quantitative determination of the target chemical substance participating in reaction (III), can be achieved by measuring a change in amount of $B_1$ [which is caused for a predetermined period of time during reaction (III)].

The third dehydrogenase can be advantageously used in order to regenerate $A_1$, so that the amount of $A_1$ to be used in the analytical reaction can be reduced. This is particularly useful when $A_1$ is an expensive compound. It is also possible to use $A_2$ alone or a mixture of $A_1$ and $A_2$, at the initiation of the reaction. For conducting reaction (III), an amount of at least one coenzyme selected from $A_1$ and $A_2$ is preferably not larger than 1/10 mole per mole of $B_1$, although the amount is not particularly limited.

In practicing the above method for quantitative determination of the present invention using a third dehydrogenase as component (5), $B_1$ is used in a concentration of 0.02 to 100 mM, preferably 0.05 to 20 mM. $A_2$ and/or $A_1$ is used in a concentration of 0.05 to 5,000 µM, preferably 5 to 500 µM. A glucose-6-phosphate dehydrogenase as the first dehydrogenase is used in a concentration of 1 to 1000 u/ml, preferably 2 to 400 u/ml. The concentration of the third dehydrogenase (u/ml) can be 20 times or more the Km value (unit: mM) thereof for $A_2$, e.g., 1 to 100 u/ml. The substrate for the third dehydrogenase can be used in a stoichiometrically excess amount, for example, 0.05 to 20 mM. The amounts of the components of the reagent for the cycling reaction can be varied depending on the type of a biological sample to be tested. The amounts exceeding the above can also be employed.

As Examples of combinations of third dehydrogenases and substrates therefor, the following combinations can be mentioned. When $A_1$ is an NAD compound or a thio-NAD compound, there can be mentioned combinations of: alcohol dehydrogenase (EC 1.1.1.1) and acetaldehyde; glycerol dehydrogenase (EC 1.1.1.6) derived from E. coli and dihydroxyacetone; L-glycerol-3-phosphate dehydrogenase (EC 1.1.1.8) derived from rabbit muscle and dihydroxyacetone phosphate; malate dehydrogenase (EC 1.1.1.37) derived from pig or bovine heart and oxaloacetic acid; and glyceraldehyde phosphate dehydrogenase (EC 1.1.1.12) derived from rabbit muscle, liver, yeast or $\underline{E}$. $\underline{coli}$ and 1,3-diphospho-D-glycerate. When $A_1$ is an NADP compound or a thio-NADP compound, there can be mentioned combinations of: glyceraldehyde phosphate dehydrogenase (EC 1.2.1.13) derived from plant chlorophyll and 1,3-diphospho-D-glycerate.

In the method of the present invention, types of coenzymes $A_1$ and $B_1$ can be appropriately selected, taking into consideration the relative activities and the like of the coenzymes, and pH conditions for the forward and reverse reactions can also be appropriately selected so that the emzymatic cycling reaction proceeds efficiently.

In practicing the method for the quantitative determination of D-glucose-6-phosphate in a biological sample by using the analytical reagent of the present invention, 0.001 to 0.5 ml of the biological sample can be added to an aqueous composition containing the above-defined components (1) to (3), (1) to (4), or (1) to (3) and (5), and the resultant solution is reacted at about 37 °C. Then, a change in absorbance at a wavelength specific for coenzyme $A_2$ or $B_1$ is measured, which is observed with respect to the reaction mixture, as between predetermined two time points during the reaction (e.g., between 3 and 4 minutes after the start of the reaction, or between 3 and 8 minutes after the start of the reaction). The period between such two time points during the reaction can be varied in the range from several minutes to several tens of minutes, depending on the type of a biological sample and the type of a target chemical substance contained therein. For example, when $A_2$ is a thio-NADH compound and $B_1$ is an NADH compound, either the produced $A_2$ is determined in terms of the change in absorbance at 400 nm, or the consumed $B_1$ is determined in terms of the change in absorbance at 340 nm. The thus obtained change in absorbance reflecting the amount of the target chemical substance is applied to a calibration curve which has been prepared with respect to standard samples containing D-glucose-6-phosphate in different concentrations, to thereby determine the amount of the target chemical-substance. By the method of the present invention, it becomes possible to realize a real-time quantitative determination of D-glucose-6-phosphate in a biological sample.

Furthermore, the method of the present invention is so designed that the target chemical substance (i.e., D-glucose-6-phosphate) itself participates in the enzymatic cycling reaction. Therefore, the determination of the target chemical substance of the method of the present invention is less influenced by other compounds present in the sample, so that the desired determination can be easily, simply done by rate assay without requiring any blank assay of the sample. With respect to the determination of $A_2$ or $B_1$, other known methods can be used instead of the measurement of absorbances described above.

Best Mode for Carrying Out the Invention

Hereinbelow, the present invention will be illustrated with reference to the following examples, which however should not be construed as limiting the present invention.

Example 1 Quantitative determination of D-glucose-6-phosphate

| Reagent | |
|---|---|
| 50 mM | Tris-HCl buffer (pH 7.1) |
| 1 mM | thio-NADP (manufactured by Sigma Co., Ltd., U.S.A.) |
| 0.5 mM | reduced NADP (manufactured by Oriental Yeast Co., Ltd., Japan) |
| 5 mM | EDTA |
| 38 u/ml | glucose-6-phosphate dehydrogenase (derived from bakers' yeast and manufactured by Sigma Co., Ltd., U.S.A.) |

Procedure

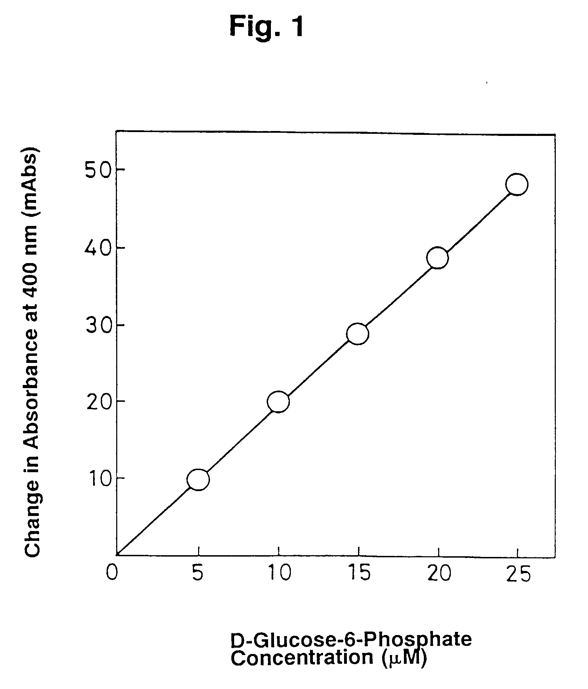

1 ml of the above reagent was placed in each of six cuvettes. To the respective cuvettes were individually added 50 μl each of six types of aqueous D-glucose-6-phosphate solutions respectively having concentrations of 0, 5, 10, 15, 20 and 25 μM. The resultant mixtures in the respective cuvettes were individually allowed to react at 37 °C. With respect to each of respective samples taken from the reaction mixtures in the six cuvettes, absorbances at 400 nm were measured 2 minutes and 5 minutes after the start of the reaction, and a change in absorbance as between the above two time points was calculated with respect to each of the samples. Results are shown in Fig. 1, which demonstrates the presence of good linearity in the relationship between the change (difference) in absorbance (with respect to the reaction mixture, as between 2 and 3 minutes after the start of the reaction) and the D-glucose-6-phosphate concentration.

Example 2 Quantitative determination of D-glucose-6-phosphate

| Reagent | |
|---|---|
| 50 mM | Tris-HCl buffer (pH 7.1) |
| 1 mM | thio-NADP (manufactured by Sigma Co., Ltd., U.S.A.) |
| 2 mM | reduced NADP (manufactured by Oriental Yeast Co., Ltd., Japan) |
| 2 mM | EDTA |
| 84.4 u/ml | glucose-6-phosphate dehydrogenase (derived from Leuconostoc mesenteroides and manufactured by Toyobo Co., Ltd., Japan) |

Procedure

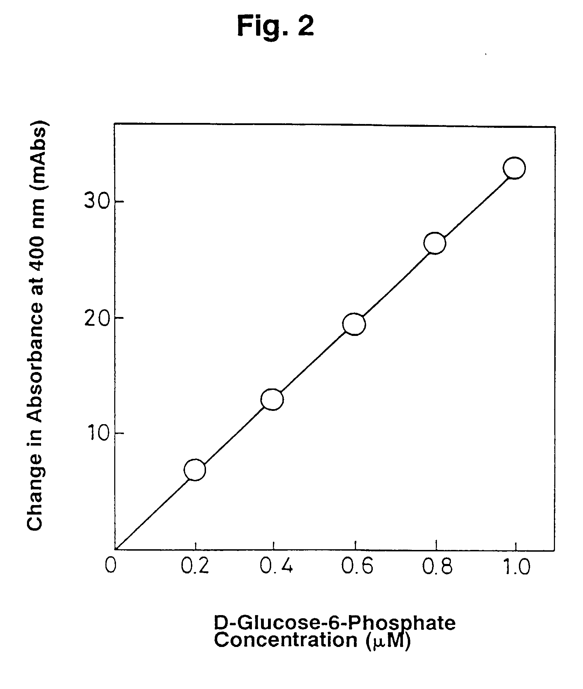

1 ml of the above reagent was placed in each of six cuvettes. To the respective cuvettes were individually added 20 µl each of six types of aqueous D-glucose-6-phosphate solutions respectively having concentrations of 0, 0.2, 0.4, 0.6, 0.8 and 1.0 µM. The resultant mixtures in the respective cuvettes were individually allowed to react at 37 °C. With respect to each of respective samples taken from the reaction mixtures in the six cuvettes, absorbances at 400 nm were measured 2 minutes and 4 minutes after the start of the reaction, and a change in absorbance as between the above two time points was calculated with respect to each of the samples. Results are shown in Fig. 2, which demonstrates the presence of good linearity in the relationship between the change (difference) in absorbance (with respect to the reaction mixture, as between 2 and 4 minutes after the start of the reaction) and the D-glucose-6-phosphate concentration.

Example 3 Quantitative determination of D-glucose-6-phosphate

| Reagent | |
|---|---|
| 50 mM | MES buffer (pH 6.5) |
| 0.5 mM | thio-NADP (manufactured by Sigma Co., Ltd., U.S.A.) |
| 2 mM | reduced NADP (manufactured by Oriental Yeast Co., Ltd., Japan) |
| 2 mM | EDTA |
| 24.8 u/ml | glucose-6-phosphate dehydrogenase (derived from Bacillus sp. and manufactured by Toyo Jozo Kabushiki Kaisha, Japan) |

Procedure

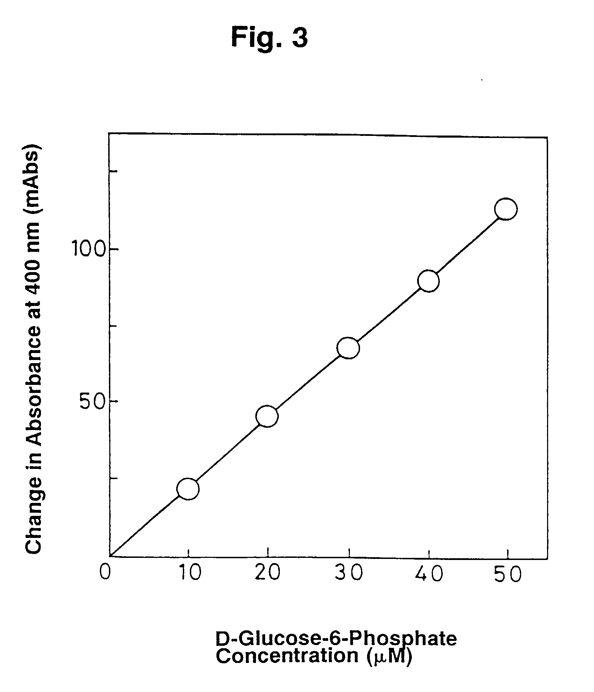

1 ml of the above reagent was placed in each of six cuvettes. To the respective cuvettes were individually added 20 µl each of six types of aqueous D-glucose-6-phosphate solutions respectively having concentrations of 0, 10, 20, 30, 40 and 50 µM. The resultant mixtures in the respective cuvettes were individually allowed to react at 37 °C. With respect to each of respective samples taken from the reaction mixtures in the six cuvettes, absorbances at 400 nm were measured 2 minutes and 4 minutes after the start of the reaction, and a change in absorbance as between the above two time points was calculated with respect to each of the samples. Results are shown in Fig. 3, which demonstrates the presence of good linearity in the relationship between the change (difference) in absorbance (with respect to the reaction mixture, as between 2 and 4 minutes after the start of the reaction) and the D-glucose-6-phosphate concentration.

Example 4 Quantitative determination of D-fructose

| Reagent (I) | |
|---|---|
| 50 mM | MES buffer (pH 7.0) |
| 0.5 mM | thio-NADP (manufactured by Sigma Co., Ltd., U.S.A.) |

(continued)

| Reagent (I) | |
|---|---|
| 2 mM | magnesium chloride |
| 0.5 mM | ATP |
| 3.6 u/ml | phosphoglucose isomerase (derived from yeast and manufactured by Boehringer-Mannheim GmbH, Germany) |
| 24.8 u/ml | glucose-6-phosphate dehydrogenase (derived from Bacillus sp. and manufactured by Toyo Jozo Kabushiki Kaisha, Japan) |

| Reagent (II) | |
|---|---|
| 50 mM | MES buffer (pH 7.0) |
| 20 mM | reduced NADP (manufactured by Oriental Yeast Co., Ltd., Japan) |
| 50 mM | EDTA |

Procedure

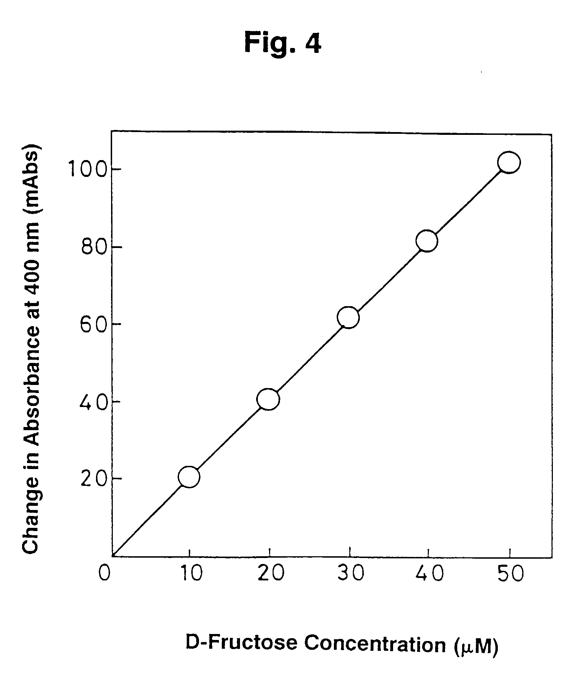

0.9 ml of the above reagent (I) was placed in each of six cuvettes. To the respective cuvettes were individually added 20 µl each of six types of aqueous D-fructose solutions respectively having concentrations of 0, 10, 20, 30, 40 and 50 µM. The resultant mixtures in the respective cuvettes were individually allowed to react at 37 °C. 5 Minutes after the start of the reaction, to each of the cuvettes was added 0.1 ml of the above reagent (II), to thereby initiate an enzymatic cycling reaction. With respect to each of respective samples taken from the reaction mixtures in the six cuvettes, absorbances at 400 ml were measured 2 minutes and 5 minutes after the addition of reagent (II), and a change in absorbance as between the above two time points was calculated with respect to each of the samples. Results are shown in Fig. 4, which demonstrates the presence of good linearity in the relationship between the change (difference) in absorbance [with respect to the reaction mixture, as between 2 and 4 minutes after the addition of reagent (II)] and the D-fructose concentration.

Industrial Applicability

According to the determination method of the present invention, an error in the quantitative determination of a target chemical substance can be minimized since two types of coenzymes exhibiting absorbances at different absorption wavelengths are used. Further, the sensitivity of the determination method can be greatly increased due to the utilization of the enzymatic cycling reaction. Thus, the method of the present invention ensures rapidness and accuracy in the determination of a target chemical substance, i.e., D-glucose-6-phosphate, even with the use of a small quantity of a biological sample.

**Claims**

1. A method for the quantitative determination of D-glucose-6-phosphate, which comprises:
reacting a biological sample containing D-glucose-6-phosphate with a reagent comprising:

(1) a glucose-6-phosphate dehydrogenase which catalyzes the reversible reaction producing D-glucono-$\delta$-lactone-6-phosphate from D-glucose-6-phosphate as a substrate in the presence of (i) a first coenzyme selected from the group consisting of thionicotinamide adenine dinucleotide phosphate or its analogue (thio-NADP compound) and thionicotinamide adenine dinucleotide or its analogue (thio-NAD compound) and (ii) a second coenzyme selected from the group consisting of nicotinamide adenine dinucleotide phosphate or its analogue (NADP compound) and nicotinamide adenine dinucleotide or its analogue (NAD compound);
(2) $A_1$ (defined hereinbelow); and
(3) $B_1$ (defined hereinbelow):

said components (1), (2) and (3) participating in the following cycling reaction:

A₁   Glucose-6-
phosphate
Dehydrogenase                    A₂

D-Glucose-6-phosphate ⇌ D-Glucono-δ-lactone-6-phosphate

B₂                                    B₁

wherein $A_1$ is a thio-NADP compound, a thio-NAD compound, an NADP compound or an NAD compound; $A_2$ is a reduced form of $A_1$; $B_1$ is a reduced NADP compound or a reduced NAD compound when $A_1$ is a thio-NADP compound or a thio-NAD compound, or a reduced thio-NADP compound or a reduced thio-NAD compound when $A_1$ is an NADP compound or an NAD compound; and $B_2$ is an oxidized form of $B_1$,

thereby effecting said cycling reaction; and

measuring a change in amount of $A_2$ or $B_1$.

2. The method according to claim 1, wherein said thio-NADP compound is thionicotinamide adenine dinucleotide phosphate (thio-NADP) or thionicotinamide hypoxanthine dinucleotide phosphate.

3. The method according to claim 1, wherein said thio-NAD compound is thionicotinamide adenine dinucleotide (thio-NAD) or thionicotinamide hypoxanthine dinucleotide.

4. The method according to claim 1, wherein said NADP compound is selected from the group consisting of nicotinamide adenine dinucleotide phosphate (NADP), acetylpyridine adenine dinucleotide phosphate (acetyl-NADP), acetylpyridine hypoxanthine dinucleotide phosphate and nicotinamide hypoxanthine dinucleotide phosphate (deamino-NADP).

5. The method according to claim 1, wherein said NAD compound is selected from the group consisting of nicotinamide adenine dinucleotide (NAD), acetylpyridine adenine dinucleotide (acetyl-NAD), acetylpyridine hypoxanthine dinucleotide and nicotinamide hypoxanthine dinucleotide (deamino-NAD).

6. An analytical reagent for use in the quantitative determination of D-glucose-6-phosphate, which comprises:

(1) a glucose-6-phosphate dehydrogenase which catalyzes the reversible reaction producing D-glucono-δ-lactone-6-phosphate from D-glucose-6-phosphate as a substrate in the presence of (i) a first coenzyme selected from the group consisting of thionicotinamide adenine dinucleotide phosphate or its analogue (thio-NADP compound) and thionicotinamide adenine dinucleotide or its analogue (thio-NAD compound) and (ii) a second coenzyme selected from the group consisting of nicotinamide adenine dinucleotide phosphate or its analogue (NADP compound) and nicotinamide adenine dinucleotide or its analogue (NAD compound);
(2) $A_1$ (defined hereinbelow); and
(3) $B_1$ (defined hereinbelow):

said components (1), (2) and (3) participating in the following cycling reaction:

wherein $A_1$ is a thio-NADP compound, a thio-NAD compound, an NADP compound or an NAD compound; $A_2$ is a reduced form of $A_1$; $B_1$ is a reduced NADP compound or a reduced NAD compound when $A_1$ is a thio-NADP compound or a thio-NAD compound, or a reduced thio-NADP compound or a reduced thio-NAD compound when $A_1$ is an NADP compound or an NAD compound; and $B_2$ is an oxidized form of $B_1$.

**Patentansprüche**

1. Verfahren zur quantitativen Bestimmung von D-Glucose-6-phosphat, umfassend:
   Umsetzen einer biologischen Probe, die D-Glucose-6- phosphat enthält, mit einem Reagenz, umfassend:

   (1) eine Glucose-6-phosphatdehydrogenase, die die reversible Reaktion katalysiert, bei der D-Glucono-δ-lacton-6-phosphat aus D-Glucose-6-phosphat als einem Substrat hergestellt wird, in Gegenwart von

   (i) einem ersten Coenzym, das aus der Gruppe ausgewählt wird, die aus Thionicotinamidadenindinucleotidphosphat oder seinem Analogon (der Thio-NADP-Verbindung) und Thionicotinamidadenindinucleotid oder seinem Analogon (der Thio-NAD-Verbindung) besteht, und
   (ii) einem zweiten Coenzym, das aus der Gruppe ausgewählt wird, die aus Nicotinamidadenindinucleotidphosphat oder seinem Analogon (der NADP-Verbindung) und Nicotinamidadenindinucleotid oder seinem Analogon (der NAD-Verbindung) besteht;

   (2) $A_1$ (hierin nachstehend definiert) ; und

   (3) $B_1$ (hierin nachstehend definiert): wobei die Komponenten (1), (2) und (3) an der folgenden Kreislaufreaktion teilnehmen:

worin $A_1$ eine Thio-NADP-Verbindung, eine Thio-NAD-Verbindung, eine NADP-Verbindung oder eine NAD-Verbindung ist; $A_2$ eine reduzierte Form von $A_1$ ist; $B_1$ eine reduzierte NADP-Verbindung oder eine reduzierte NAD-Verbindung ist, wenn $A_1$ eine Thio-NADP-Verbindung oder eine Thio-NAD-Verbindung ist, oder eine reduzierte Thio-NADP-Verbindung oder eine reduzierte Thio-NAD-Verbindung ist, wenn $A_1$ eine NADP-Verbindung oder eine NAD-Verbindung ist; und $B_2$ eine oxidierte Form von $B_1$ ist;

wodurch die Kreislaufreaktion bewirkt wird; und
Messen einer Veränderung der Menge von $A_2$ oder $B_1$.

2. Verfahren nach Anspruch 1, worin die Thio-NADP-Verbindung Thionicotinamidadenindinucleotidphosphat (Thio-NADP) oder Thionicotinamidhypoxanthindinucleotidphosphat ist.

3. Verfahren nach Anspruch 1, worin die Thio-NAD-Verbindung Thionicotinamidadenindinucleotid (Thio-NAD) oder Thionicotinamidhypoxanthindinucleotid ist.

4. Verfahren nach Anspruch 1, worin die NADP-Verbindung aus der Gruppe ausgewählt wird, die aus Nicotinamidadenindinucleotidphosphat (NADP), Acetylpyridinadenindinucleotidphosphat (Acetyl-NADP), Acetylpyridinhypoxanthindinucleotidphosphat und Nicotinamidhypoxanthindinucleotidphosphat (Deamino-NADP) besteht.

5. Verfahren nach Anspruch 1, worin die NAD-Verbindung aus der Gruppe ausgewählt wird, die aus Nicotinamidadenindinucleotid (NAD), Acetylpyridinadenindinucleotid (Acetyl-NAD), Acetylpyridinhypoxanthindinucleotid und Nicotinamidhypoxanthindinucleotid (Deamino-NAD) besteht.

6. Analytisches Reagenz zur Verwendung bei der quantitativen Bestimmung von D-Glucose-6-phosphat, umfassend:

   (1) eine Glucose-6-phosphatdehydrogenase, die die reversible Reaktion katalysiert, bei der D-Glucono-δ-lacton-6-phosphat aus D-Glucose-6-phosphat als einem Substrat hergestellt wird, in Gegenwart von

      (i) einem ersten Coenzym, das aus der Gruppe ausgewählt wird, die aus Thionicotinamidadenindinucleotidphosphat oder seinem Analogon (der Thio-NADP-Verbindung) und Thionicotinamidadenindinucleotid oder seinem Analogon (der Thio-NAD-Verbindung) besteht, und
      (ii) einem zweiten Coenzym, das aus der Gruppe ausgewählt wird, die aus Nicotinamidadenindinucleotidphosphat oder seinem Analogon (der NADP-Verbindung) und Nicotinamidadenindinucleotid oder seinem Analogon (der NAD-Verbindung) besteht;

   (2) $A_1$ (hierin nachstehend definiert); und

   (3) $B_1$ (hierin nachstehend definiert): wobei die Komponenten (1), (2) und (3) an der folgenden Kreislaufreaktion teilnehmen:

worin $A_1$ eine Thio-NADP-Verbindung, eine Thio-NAD-Verbindung, eine NADP-Verbindung oder eine NAD-Verbindung ist; $A_2$ eine reduzierte Form von $A_1$ ist; $B_1$ eine reduzierte NADP-Verbindung oder eine reduzierte NAD-Verbindung ist, wenn $A_1$ eine Thio-NADP-Verbindung oder eine Thio-NAD-Verbindung ist, oder eine reduzierte Thio-NADP-Verbindung oder eine reduzierte Thio-NAD-Verbindung ist, wenn $A_1$ eine NADP-Ver-

bindung oder eine NAD-Verbindung ist; und $B_2$ eine oxidierte Form von $B_1$ ist.

**Revendications**

1. Procédé de détermination quantitative du D-glucose-6-phosphate qui comprend:
   à faire réagir un échantillon biologique contenant du D-glucose-6-phosphate sur un réactif comprenant :

   (1) une glucose-6-phosphate déshydrogénase qui catalyse la réaction réversible produisant du D-glucono-δ-lactone-6-phosphate à partir du D-glucose-6-phosphate comme substrat en la présence de (i) une première coenzyme choisie dans le groupe consistant en le thionicotinamide adénine dinucléotide phosphate ou son analogue (thio-NADP) et le thionicotinamide adénine dinucléotide ou son analogue (thio-NAD) et (ii) une seconde coenzyme choisie dans le groupe consistant en le nicotinamide adénine dinucléotide phosphate ou son analogue (NADP) et le nicotinamide adénine dinucléotide ou son analogue (NAD);
   (2) $A_1$ (défini ci-dessous) ; et
   (3) $B_1$ (défini ci-dessous):

   les constituants (1), (2) et (3) participant à la réaction suivante de cyclisation

   dans laquelle $A_1$ est un thio-NADP, un thio-NAD, un NADP ou un NAD; $A_2$ est une forme réduite de $A_1$ ; $B_1$ est un NADP réduit ou un NAD réduit lorsque $A_1$ est un thio-NADP ou est un thio-NAD ou un thio-NADP réduit ou un thio-NAD réduit lorsque $A_1$ est un NADP ou un NAD ; et $B_2$ est une forme oxydée de $B_1$, effectuant ainsi la réaction de cyclisation ; et
   à mesurer la variation de quantité de $A_2$ ou de $B_1$.

2. Procédé suivant la revendication 1, dans lequel le thio-NADP est le thionicotinamide adénine dinucléotide phosphate (thio-NADP) ou le thionicotinamide hypoxanthine dinucléotide phosphate.

3. Procédé suivant la revendication 1, dans lequel le thio-NAD est le thionicotinamide adénine dinucléotide (thio-NAD) ou le thionicotinamide hypoxanthine dinucléotide.

4. Procédé suivant la revendication 1, dans lequel le NADP est choisi dans le groupe consistant en le nicotinamide adénine dinucléotide phosphate (NADP), l'acétylpyridine adénine dinucléotide phosphate (acétyl-NADP), l'acétylpyridine hypoxanthine dinucléotide phosphate et le nicotinamide hypoxanthine dinucléotide phosphate (déamino-NADP).

5. Procédé suivant la revendication 1, dans lequel le NAD est choisi dans le groupe consistant en le nicotinamide adénine dinucléotide (NAD), l'acétylpyridine adénine dinucléotide (acétyle-NAD), acétylpyridine hypoxanthine dinucléotide et le nicotinamide hypoxantine dinucléotide (déamino-NAD).

**6.** Réactif analytique destiné à être utilisé dans la détermination quantitative du D-glucose-6-phosphate, qui comprend :

(1) une glucose-6-phosphate déshydrogénase qui catalyse la réaction réversible produisant du D-glucono-δ-lactone-6-phosphate à partir du D-glucose-6-phosphate comme substrat en la présence de (i) une première coenzyme choisie dans le groupe consistant en le thionicotinamide adénine dinucléotide phosphate ou son analogue (thio-NADP) et le thionicotinamide adénine dinucléotide ou son analogue (thio-NAD) et (ii) une seconde coenzyme choisie dans le groupe consistant en le nicotinamide adénine dinucléotide phosphate ou son analogue (NADP) et le nicotinamide adénine dinucléotide ou son analogue (NAD);

(2) $A_1$ (défini ci-dessous) ; et

(3) $B_1$ (défini ci-dessous):

les constituants (1), (2) et (3) participant à la réaction suivante de cyclisation

dans laquelle $A_1$ est un thio-NADP, un thio-NAD, un NADP ou un NAD; $A_2$ est une forme réduite de $A_1$ ; $B_1$ est un NADP réduit ou un NAD réduit lorsque $A_1$ est un thio-NADP ou est un thio-NAD ou un thio-NADP réduit ou un thio-NAD réduit lorsque $A_1$ est un NADP ou un NAD ; et $B_2$ est une forme oxydée de $B_1$,

# Fig. 1

D-Glucose-6-Phosphate
Concentration (μM)

# Fig. 2

# Fig. 3

Change in Absorbance at 400 nm (mAbs) vs. D-Glucose-6-Phosphate Concentration (μM)

# Fig. 4